(19) Europäisches Patentamt / European Patent Office / Office européen des brevets

(11) EP 1 824 866 B1

(12) **EUROPÄISCHE PATENTSCHRIFT**

(45) Veröffentlichungstag und Bekanntmachung des Hinweises auf die Patenterteilung:
**06.10.2010 Patentblatt 2010/40**

(21) Anmeldenummer: **05815439.4**

(22) Anmeldetag: **18.11.2005**

(51) Int Cl.:
*C07F 9/02* (2006.01) *C07F 9/09* (2006.01)
*C07F 9/40* (2006.01) *C07F 9/32* (2006.01)

(86) Internationale Anmeldenummer:
**PCT/EP2005/012400**

(87) Internationale Veröffentlichungsnummer:
**WO 2006/063655 (22.06.2006 Gazette 2006/25)**

(54) **VERFAHREN ZUR HERSTELLUNG VON ONIUM-SALZEN MIT DIALKYLPHOSPHAT-, DIALKYLPHOSPHINAT- ODER (O-ALKYL)-ALKYL- ODER ALKYL-PHOSPHONAT-ANIONEN MIT GERINGEM HALOGENID-GEHALT**

METHOD FOR PRODUCING ONIUM SALTS COMPRISING DIALKYLPHOSPHATE ANIONS, DIALKYLPHOSPHINATE ANIONS OR (O-ALKYL)-ALKYL ANIONS OR ALKYL-PHOSPHONATE ANIONS HAVING A LOW HALIDE CONTENT

PROCEDE POUR PRODUIRE DES SELS D'ONIUM COMPRENANT DES ANIONS DE DIALKYLPHOSPHATE, DE DIALKYLPHOSPHINATE, OU D'ALKYLE (O-ALKYLE) OU D'ALKYLE-PHOSPHONATE PRESENTANT UNE TENEUR FAIBLE EN HALOGENURE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **14.12.2004 DE 102004060075**

(43) Veröffentlichungstag der Anmeldung:
**29.08.2007 Patentblatt 2007/35**

(60) Teilanmeldung:
**09014638.2 / 2 180 002**

(73) Patentinhaber: **Merck Patent GmbH**
**64293 Darmstadt (DE)**

(72) Erfinder:
- **IGNATYEV, Nikolai (Mykola)**
  **47058 Duisburg (DE)**
- **WELZ-BIERMANN, Urs**
  **64646 Heppenheim (DE)**
- **KUCHERYNA, Andriy**
  **42117 Wuppertal (DE)**
- **WILLNER, Helge**
  **45481 Muelheim/Ruhr (DE)**

(56) Entgegenhaltungen:
**WO-A-03/087110** **WO-A-2004/106288**
**WO-A1-2004/106287** **WO-A1-2004/106287**

- **SUTTER P ET AL: "THE SPECIFICITY OF REDUCTIVE DECHLORINATION IN THE POLYCHLOROPYRIDINE SERIES. SYNTHESIS OF 2,3,5-TRICHLORO- AND OF 2,3,5,6-TETRACHLOROPYRIDINE" JOURNAL OF HETEROCYCLIC CHEMISTRY, HETEROCORPORATION. PROVO, US, Bd. 17, Mai 1980 (1980-05), Seiten 493-496, XP000960739 ISSN: 0022-152X**
- **DATABASE BEILSTEIN [Online] BEILSTEIN INSTITUTE FOR ORGANIC CHEMISTRY, FRANKFURT-MAIN, DE; XP002365775 Database accession no. Reaction ID 9306032 & CAMEREL, F. ET AL.: CHEM. COMMUN., Bd. 15, 2003, Seiten 1958-1959,**
- **DATABASE BEILSTEIN [Online] BEILSTEIN INSTITUTE FOR ORGANIC CHEMISTRY, FRANKFURT-MAIN, DE; XP002365776 Database accession no. Reaction ID 8937018 & ZAKHAROV, N.A.: RUSS. J. PHYS. CHEM. (EN(, Bd. 75, Nr. 11, 2001, Seiten 1924-1927,**
- **D.E.C. Corbridge: "Phosphorus: An Outline of its Chemistry, Biochemistry and Technology 2nd ed." 1980, Elsevier , page 247**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- EMELEUS H J ET AL: "ORGANOMETALLIC AND ORGANOMETALLOIDAL FLUORINE COMPOUNDS. PART XII. BISTRIFLUOROMETHYLPHOSPHINIC ACID AND RELATED PHOSPHORUS OXY-ACIDS" JOURNAL OF THE CHEMICAL SOCIETY, CHEMICAL SOCIETY, LETCHWORTH; GB LNKD-DOI:10.1039/JR9550000563, 1 January 1955 (1955-01-01), pages 563-574, XP009078915 ISSN: 0368-1769
- CORBRIDGE D.E.C.: 'Phosphorus: An Outline of its Chemistry, Biochemistry and Technology 2nd ed.', 1980, ELSEVIER Seite 247

## Beschreibung

Die Erfindung betrifft ein Verfahren zur Herstellung von Onium-Salzen mit Dialkylphosphat-, Anionen durch Umsetzung eines Onium-Halogenids mit einem Phosphorsäuretrialkylester, einem Trialkylsilylester oder einem gemischten Alkyl-trialkylsilylester der Phosphor säure.

Eine große Anzahl von Onium-Salzen, auch Dialkylphosphate, sind als ionische Flüssigkeiten verwendbar, Ionische Flüssigkeiten stellen durch Ihre Eigenschaften in der modernen Forschung eine wirksame Alternative zu traditionellen flüchtige organischen Solventien für die organische Synthese dar. Die Verwendung von ionischen Flüssigkeiten als neues Reaktionsmedium könnte weiterhin eine praktische Lösung sowohl für die Lösungsmittel Emission als auch für Probleme in der Wiederaufbereitung von Katalysatoren sein.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Der Schmelzpunkt kann jedoch auch höher liegen, ohne die Anwendbarkeit der Salze auf allen Anwendungsgebieten zu beschränken. Beispiele für organische Kationen sind unter anderem Tetraalkylammonium, Tetraalkylphosphonium, N-Alkylpyridinium, 1,3-Dialkylimidazolium oder Trialkylsulfonium. Unter einer Vielzahl von geeigneten Anionen sind beispielsweise $BF_4^-$. $PF_6^-$, $SbF_6^-$, $NO_3^-$, $CF_3SO_3^-$, $(CF_3SO_2)_2N^-$, $ArylSO_3^-$, $CF_3CO_2^-$, $CH_3CO_2^-$ oder $Al_2Cl_7^-$ zu nennen.

Eine generelle Methode zur Herstellung von Onium-Dialkylphosphaten ist beispielsweise die Alkylierung der organischen Base, d.h. beispielsweise des Amins, Phosphins, Guanidins oder der heterocyclischen Base, mit einem Phosphorsäuretrialkylestsr, auch bekannt durch D. Corbridge, Phosphorus. An Outline of its Chemistry, Biochemistry and Technology, 2nd Edition, Elsevier, New York, 1980 oder für Phosphoniumsalze, bekannt durch WO 04/094438). Eine generelle Methode zur Herstellung von Onium-Dialkylphosphinaten ist durch Jean, Bull. Soc. Chim. Fr. (1957), 783-785 oder R. Jentzsch et al, J. Prakt. Chem. (1977), 319, 871-874 bekannt Ein Nachteil dieser Methoden ist jedoch, dass ein Substituent des entstehenden Onium-Kations immer der entsprechenden Alkylgruppe des Alkylesters entspricht. Setzt man beispielsweise 1-Butylimidazolium mit Trimethylphosphat um, so entsteht 1-Butyl-3-methylimidazolium dimethylphosphat. Erwünscht sind jedoch unsymmetrisch substituierte Onium-Sabe, d.h. Salze, bei denen die Alkylgruppe des eingesetzten Esters nicht Substituent des entstehenden Onium-Salzes wird.

WO 2004/106288 beschreibt die Herstellung von Guanidiniumsalzen mit speziellen Anionen, umfassend Bis (perfluoralkyl)phosphinat oder Perfluoralkylphosphonat durch Umsetzung eines Guanidiniumsalzes mit einem Anion ausgewählt aus der Gruppe $F^-$, $Cl^-$, $Br^-$, $I^-$, $[HF_2]^-$, $[CN]^-$, $[SCN]^-$, $[CH_3COO]^-$, $[CF_3COO]^-$, $[CF_3SO_3]^-$, $[CH_3OSO_3]^-$, $[SiF_6]^{2-}$, $[BF_4]^-$, $[SO_4]^{2}$-, $[NO_3]^-$, Tosylate, Malonate, substituierte Malonate und $[CO_3]^-$, mit einem Ester.

Sutter et al, J. Heterocyclic Chem. 1980, 17, 43 beschreibt die Alkylierung eines Tetramethylammoniumchlorids oder Tetraethylammoniumchlorids mit einem Überschuss an Dimethylmethylphosphonat bei einer Temperatur von 150°C zur Herstellung der Ammoniumphosphonate.

Aus Zakharov N.A. et al, Russ. J. Phys. Chem. (engl trans), 75, 11,2001, 1924-1927 ist die Herstellung von Trioctylmethylammonium di(2-ethylhexyl)phosphat durch Umsalzungsreaktion aus Methyltrioctylammonium chlorid und Kalium di(2-ethylhexyl)phosphat bekannt. Das Kaliumsalz entsteht durch Umsetzung der freien Säure in wässriger KOH.

Aus Camerel, Franck et al, Chem. Commun. 15, 2003, 1958-1959 ist die Synthese eines speziellen Onium dihexadecylphosphats durch Umsalzungsreaktion aus dem speziellen Oniumiodid und Natriumdihexadecylphosphat. Das Natriumsalz entsteht intermediär durch Reaktion der freien Di(hexydecyl)phosphorsäure mit der wässrigen NaOH-Lösung.

Unsymmetrische Onium-Salze mit Dialkylphosphat-, Dialkylphosphinat-, (O-Alkyl)-alkyl- oder Alkyl-phosphonat-Anionen, wie zuvor definiert, können auch durch eine Metathese hergestellt werden, indem ein Oniumhalogenid mit einem entsprechenden Alkalimetallsalz der entsprechenden Säure umgesetzt würde. Das anfallende Alkalimetallhalogenid, z.B. Natriumchlorid, muss jedoch durch eine zusätzliche Aufreinigungsmethode entfernt werden. Die Verunreinigung durch Halogenid-Ionen, beispielsweise Chlorid-Ionen, größer als 1000 ppm (0,1 %), reduziert die Anwendbarkeit der ionischen Flüssigkeit, insbesondere in der Anwendung für elektrochemische Prozesse. Daher ist bei Verfahren zur Herstellung von Onium-Salzen, insbesondere ionischer Flüssigkeiten, die Technologie von entscheidender Bedeutung, damit diese durch die Reaktion per se oder die Reaktionsführung mit geringen Verunreinigungen synthetisiert werden können und so weitere kostenintensive zusätzliche Verfahrensschritte bei der Synthese entfallen.

Aufgabe der vorliegenden Erfindung war dementsprechend ein alternatives Verfahren zur Herstellung von Onium-Salzen mit Dialkylphosphat-Anionen mit geringem Halogenid-Gehalt zur Verfügung zu stellen, welches zu Salzen, vorzugsweise zu unsymmetrisch substituierten Onium-Salzen, mit hoher Reinheit in guter Ausbeute führt und auch für eine großtechnische Produktion geeignet ist

Selbstverständlich ist ein solches Verfahren dann auch zur Herstellung von symmetrisch substituierten Onium-Salzen geeignet. Das erfindungsgemäße Verfahren ist ebenfalls zur Herstellung von Onium-Salzen mit Diarylphosphat, Diarylphosphinat, Aryl-phosphonat oder gemischten Alkyl-aryl-Phosphaten, -phosphinaten oder -phosphonaten geeignet. Aryl beschreibt hierbei insbesondere unsubstituiertes oder substituiertes Phenyl, wobei die Möglichkeiten der Substitution nachfolgend für Phenyl beschrieben wird und Alkyl eine für die Dialkylphosphate, beschriebene Bedeutung hat.

Die Aufgabe wird durch das erfindungsgemäße Verfahren gelöst, da der eingesetzte Ester das Anion des eingesetzten Oniumhalogenids alkyliert und nicht das organische Onium-Kation. Die als Nebenprodukt entstehenden Alkylhalogenide sind in der Regel Gase oder sehr flüchtige Verbindungen, die ohne großen prozesstechnischen Aufwand aus der Reaktionsmischung entfernt werden können. Einige dieser Nebenprodukte sind selbst wertvolle Materialien für organische Synthesen.

Gegenstand der Erfindung ist daher ein Verfahren zur Herstellung von Onium-Salzen mit Dialkylphosphat-. Anionen durch Umsetzung eines Onium-Halogenids mit einem Phosphorsäuretrialkylester, einem Trialkylsilylester oder einem gemischten Alkyl-trialkylsilylester der Phosphor säure gemäß Anspruch 1.

Geeignete Onium-Halogenide sind Phosphoniumhalogenide, Thiouroniumhalogenide, Guanidiniumhalogenide oder Halogenide mit heterocyclischem Kation, wobei die Halogenide aus der Gruppe Chloride, Bromide oder iodide ausgewählt werden können. Bevorzugt werden im erfindungsgemäßen Verfahren Chloride oder Bromide eingesetzt. Bevorzugt werden zur Herstellung von Thiouronium-Salzen Tnouroniumiodide eingesetzt.

Die Onium-Halogenide sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Phosphoniumhalogenide können beispielsweise durch die Formel (1)

$$[PR_4]^+ \, Hal^- \qquad (1),$$

beschrieben werden,
wobei
Hal Cl, Br oder I und
R jeweils unabhängig voneinander
H. wobei nicht alle Substituenten R gleichzeitig H sein dürfen, geradkettiges oder verzweigtes Alkyl mit 1-20 C-Atomen, geradkettiges oder verzweigtes Alkenyl mit 2-20 C-Atomen und einer oder mehreren Doppelbindungen,

geradkettiges oder verzweigtes Alkinyl mit 2-20 C-Atomen und einer oder mehreren Dreifachbindungen, gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise oder vollständig mit -F substituiert sein können, wobei jedoch nicht alle vier oder drei R vollständig mit F substituiert sein dürfen,
und wobei ein oder zwei nicht benachbarte und nicht α- oder ω-ständige Kohlenstoffatome des R, durch Atome und/ oder Atomgruppierungen ausgewählt aus der Gruppe -O-, -S-, -S(O)- oder $-SO_2-$ ersetzt sein können.

Ausgeschlossen sind jedoch Verbindungen der Formel (1), in denen alle vier oder drei Substituenten R vollständig mit Halogenen substituiert sind, beispielsweise Tris(trifluormethyl)methylphosphoniumchlorid, Tetra(trifluormethyl)phosphoniumchlorid oder Tetra(nonafluorbutyl)phosphoniumchlorid.

Thiouroniumhalogenide können beispielsweise durch die Formel (2)

$$[(R^1R^2N)\text{-}C(=SR^7)(NR^3R^4)]^+ \, Hal^- \qquad (2)$$

und Guanidiniumhalogenide können beispielsweise durch die Formel (3)

$$[C(NR^1R^2)(NR^3R^4)(NR^5R^6)]^+ \, Hal^- \qquad (3),$$

beschrieben werden,
wobei
Hal Cl, Br oder I und
$R^1$ bis $R^7$ jeweils unabhängig voneinander
Wasserstoff oder CN, wobei Wasserstoff für $R^7$ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen, geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten $R^1$ bis $R^7$ teilweise oder vollständig mit -F substituiert sein können, wobei jedoch nicht alle Substituenten an einem N-Atom vollständig mit F substituiert sein dürfen, wobei die Substituenten $R^1$ bis $R^7$ paarweise durch Einfach- oder Doppelbindung miteinander verbunden sein können

und wobei ein oder zwei nicht benachbarte, nicht direkt am Heteroatom gebundene und nicht ω-ständige Kohlenstoffatome der Substituenten $R^1$ bis $R^6$ durch Atome und/oder Atomgruppierungen ausgewählt aus der Gruppe -O-, -S-, -S(O)- oder $-SO_2-$ ersetzt sein können.

Halogenide mit heterocyclischem Kation können beispielsweise durch die Formel (4)

$$[HetN]^+ \; Hal^- \qquad (4)$$

beschrieben werden, wobei

Hal  Cl, Br oder I und

$HetN^+$  ein heterocyclisches Kation, ausgewählt aus der Gruppe

Imidazolium
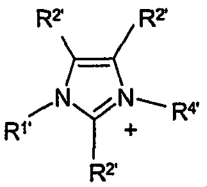
,
1H-Pyrazolium
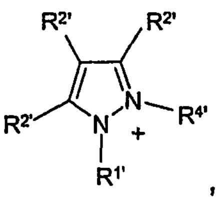
,
3H-Pyrazolium
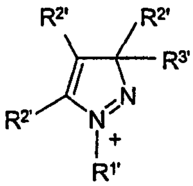
,
4H-Pyrazolium
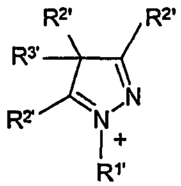
,
1-Pyrazolinium
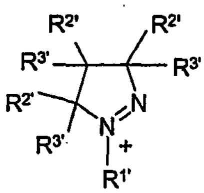
,

2-Pyrazolinium
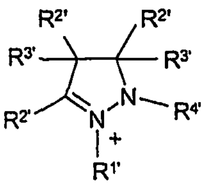
,
3-Pyrazolinium
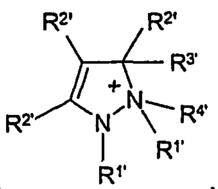
,
2,3-Dihydro-Imidazolinium
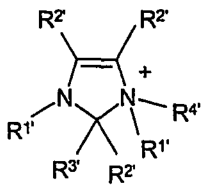
,
4,5-Dihydro-Imidazolinium
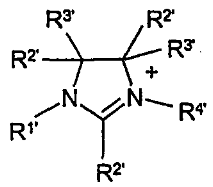
,

2,5-Dihydro-Imidazolinium
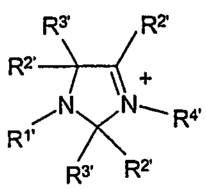
,
Pyrrolidinium
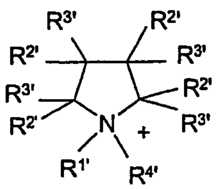
,
1,2,4-Triazolium
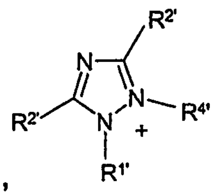
,
1,2,4-Triazolium
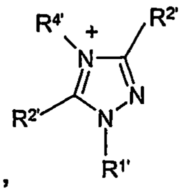
,
1,2,3-Triazolium
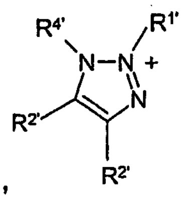
,

1,2,3-Triazolium
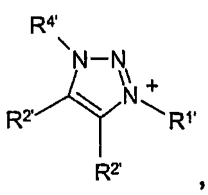
,
Pyridinium
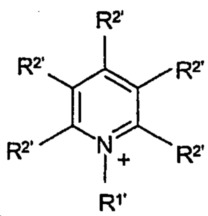
,
Pyridazinium
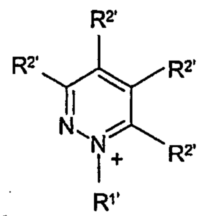
,
Pyrimidinium
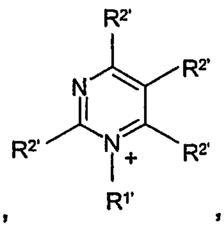
,
Piperidinium
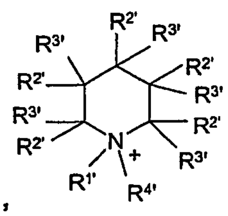
,

Morpholinium

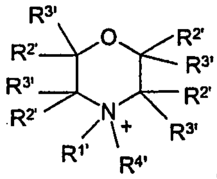

,

Pyrazinium

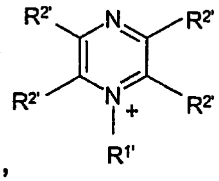

,

Thiazolium

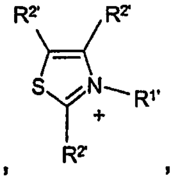

,

Oxazolium

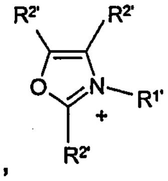

,

Indolium

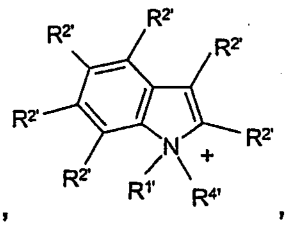

,

Chinolinium

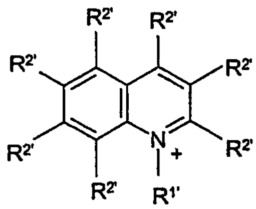

,

Isochinolinium

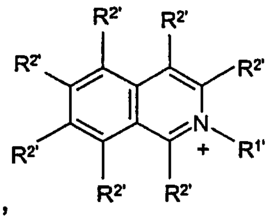

,

Chinoxalinium

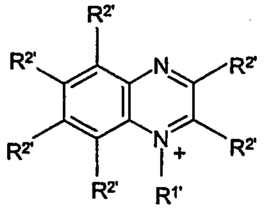

oder

Indolinium

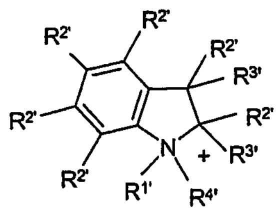

bedeutet, wobei die Substituenten
$R^{1'}$ bis $R^{4'}$ jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-$C_1$-$C_6$-alkyl bedeutet,
wobei die Substituenten $R^{1'}$ und $R^{4'}$ teilweise oder vollständig mit F substituiert sein können, wobei jedoch nicht gleichzeitig $R^{1'}$ und $R^{4'}$ CN sind oder vollständig mit F substituiert sein dürfen,
wobei die Substituenten $R^{2'}$ und $R^{3'}$ teilweise oder vollständig mit Halogenen oder teilweise mit $NO_2$ oder CN substituiert sein dürfen
und wobei ein oder zwei nicht benachbarte, nicht direkt am Heteroatom gebundene und nicht ω-ständige Kohlenstoffatome der Substituenten $R^{1'}$ bis $R^{4'}$, durch Atome und/oder Atomgruppierungen ausgewählt aus der Gruppe -O-, -S-, -S(O)- oder $-SO_2-$ ersetzt sein können.

Unter vollständig ungesättigten Substituenten werden im Sinne der vorliegenden Erfindung auch aromatische Substituenten verstanden.

Als Substituenten R und $R^1$ bis $R^7$ der Verbindungen der Formeln (1) bis (3) kommen erfindungsgemäß dabei neben Wasserstoff bevorzugt in Frage: $C_1$- bis $C_{20}$-, insbesondere $C_1$- bis $C_{14}$-Alkylgruppen, und gesättigte oder ungesättigte, d.h. auch aromatische, $C_3$- bis $C_7$-Cycloalkylgruppen, die mit $C_1$- bis $C_6$-Alkylgruppen substituiert sein können, insbesondere Phenyl. Die Substituenten R und $R^1$ bis $R^7$ können jedoch ebenfalls durch weitere funktionelle Gruppen

substituiert sein, beispielsweise durch CN, $SO_2R'$, $SO_2OR'$ oder COOR'. R' bedeutet nicht oder teilweise fluoriertes $C_1$- bis $C_6$ Alkyl, $C_3$- bis $C_7$-Cycloalkyl, unsubstituiertes oder substituiertes Phenyl.

Die Substituenten R in den Verbindungen der Formel (1) können dabei gleich oder verschieden sein. Bevorzugt sind drei Substituenten in Formel (1) gleich und ein Substituent verschieden.

Der Substituent R ist insbesondere bevorzugt Methyl, Ethyl, Isopropyl, Propyl, Butyl, sek.-Butyl, Pentyl, Hexyl, Octyl, Decyl oder Tetradecyl.

Bis zu vier Substituenten des Guanidinium-Kations $[C(NR^1R^2)(NR^3R^4)(NR^5R^6)]^+$ können auch paarweise derart verbunden sein, dass mono-, bi- oder polycyclische Kationen entstehen.

Ohne Einschränkung der Allgemeinheit sind Beispiele für solche Guanidinium-Kationen:

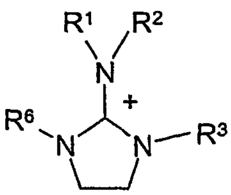 , 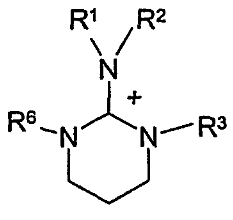 , 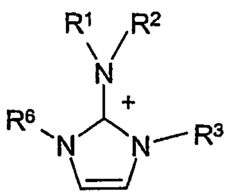 , 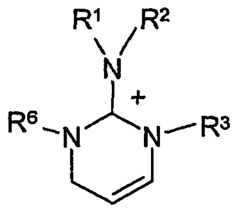 ,

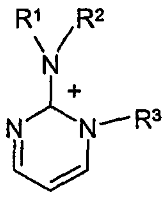 , 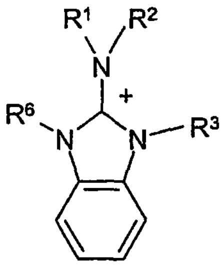 , 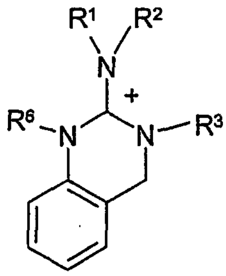 , 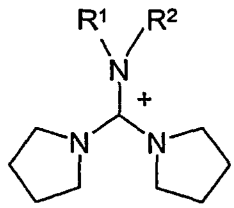

oder

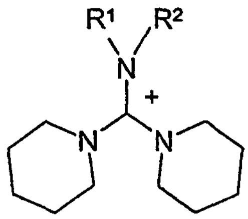 ,

wobei die Substituenten $R^1$ bis $R^3$ und $R^6$ eine zuvor angegebene oder besonders bevorzugte Bedeutung haben können.

Gegebenenfalls können die Carbocyclen oder Heterocyclen der zuvor angegebenen Guanidinium-Kationen noch durch $C_1$- bis $C_6$-Alkyl, $C_1$- bis $C_6$-Alkenyl, $NO_2$, F, Cl, Br, I, $C_1$-$C_6$-Alkoxy, $SCF_3$, $SO_2CH_3$, $SO_2CF_3$, COOR", $SO_2NR''_2$, $SO_2X'$, $SO_3R''$ oder substituiertes oder unsubstituiertes Phenyl substituiert sein, wobei X' und R" eine zuvor oder später angegebene Bedeutung haben.

Bis zu vier Substituenten des Thiouroniumkations $[(R^1R^2N)\text{-}C(=SR^7)(NR^3R^4)]^+$ können auch paarweise derart verbunden sein, dass mono-, bi- oder polycyclische Kationen entstehen. Ohne Einschränkung der Allgemeinheit sind Beispiele für solche Kationen im folgenden angegeben:

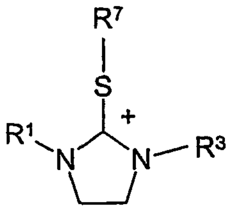,
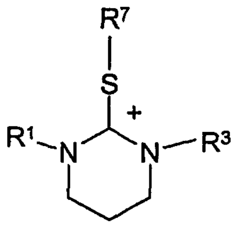,
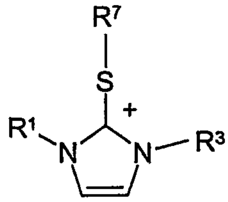,
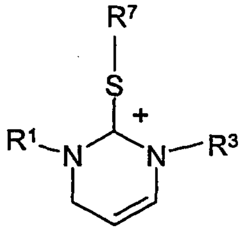,

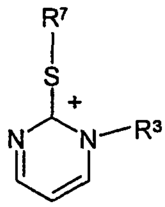,
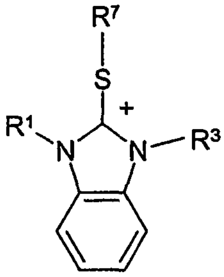,
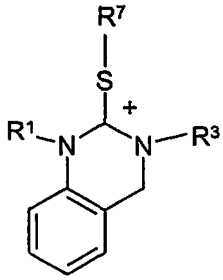,
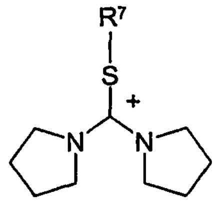

oder

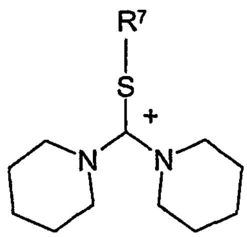,

wobei die Substituenten $R^1$, $R^3$ und $R^7$ eine zuvor angegebene oder besonders bevorzugte Bedeutung haben können. Gegebenenfalls können die Carbocyclen oder Heterocyclen der zuvor angegebenen Guanidinium-Kationen noch durch $C_1$- bis $C_6$-Alkyl, $C_1$- bis $C_6$-Alkenyl, $NO_2$, F, Cl, Br, I, $C_1$-$C_6$-Alkoxy, $SCF_3$, $SO_2CH_3$, $SO_2CF_3$, COOR", $SO_2NR''_2$, $SO_2X'$, $SO_3R''$ oder substituiertes oder unsubstituiertes Phenyl substituiert sein, wobei X' und R" eine zuvor oder später angegebene Bedeutung haben.

Die $C_1$-$C_{14}$-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl oder Tetradecyl. Gegebenenfalls perfluoriert, beispielsweise als Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Vinyl, Allyl, 2- oder 3-Butenyl, Isobutenyl, sek.-Butenyl, ferner 4-Pentenyl, iso-Pentenyl, Hexenyl, Heptenyl, Octenyl, $-C_9H_{17}$, $-C_{10}H_{19}$ bis $-C_{20}H_{39}$; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, $-C_9H_{15}$, $-C_{10}H_{17}$ bis $-C_{20}H_{37}$, vorzugsweise Ethinyl, 1- oder 2-Propinyl, 2- oder 3-Butinyl, 4-Pentinyl, 3-Pentinyl oder Hexinyl.

Aryl-$C_1$-$C_6$-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit Halogenen, insbesondere -F und/oder -Cl, oder teilweise mit $-NO_2$, substituiert sein können, besonders bevorzugt Benzyl oder Phenylpropyl. Der Phenylring oder auch die Alkylenkette können jedoch ebenfalls durch weitere funktionelle Gruppen substituiert sein, beispielsweise durch CN, $SO_2R'$, $SO_2OR'$ oder COOR', mit R' = nicht oder teilweise fluoriertes $C_1$- bis $C_6$-Alkyl, $C_3$- bis $C_7$-Cycloalkyl, unsubstituiertes oder substituiertes Phenyl.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit $C_1$- bis $C_6$-Alkylgruppen substituiert sein können, wobei wiederum die Cycloalkylgruppe oder die mit $C_1$- bis $C_6$-Alkylgruppen substituierte Cycloalkylgruppe auch mit Halogenatomen wie F, Cl, Br oder I, insbesondere F oder Cl oder $NO_2$ substituiert sein kann. Die Cycloalkylgruppen können jedoch ebenfalls durch weitere funktionelle Gruppen substituiert sein, beispielsweise durch CN, $SO_2R'$, $SO_2OR'$ oder COOR'. R' hat dabei eine zuvor definierte Bedeutung.

In den Substituenten R, $R^1$ bis $R^6$ oder $R^{1'}$ bis $R^{4'}$ können auch ein oder zwei nicht benachbarte und nicht $\alpha$-ständig zum Heteroatom oder $\omega$gebundene gebundene Kohlenstoffatome, durch Atome und/oder Atomgruppierungen ausgewählt aus der Gruppe -O-, -S-, -S(O)- oder $-SO_2$-ersetzt werden.

Ohne Einschränkung der Allgemeinheit sind Beispiele für derart modifizierte Substituenten R, $R^1$ bis $R^6$ und $R^{1'}$ bis $R^{4'}$:

$-OCH_3$, $-OCH(CH_3)_2$, $-CH_2OCH_3$, $-CH_2-CH_2-O-CH_3$, $-C_2H_4OCH(CH_3)_2$,

$-C_2H_4SC_2H_5$, $-C_2H_4SCH(CH_3)_2$, $-S(O)CH_3$, $-SO_2CH_3$, $-SO_2C_6H_5$, $-SO_2C_3H_7$,

$-SO_2CH(CH_3)_2$, $-SO_2CH_2CF_3$, $-CH_2SO_2CH_3$, $-O-C_4H_8-O-C_4H_9$, $-CF_3$, $-C_2F_5$,

$-C_3F_7$, $-C_4F_9$, $-CF_2CF_2H$, $-CF_2CHFCF_3$, $-CF_2CH(CF_3)_2$, $-C_2F_4N(C_2F_5)C_2F_5$,

$-CHF_2$, $-CH_2CF_3$, $-C_2F_2H_3$, $-C_3FH_6$, $-CH_2C_3F_7$, $-CH_2C(O)OCH_3$, $-CH_2C_6H_5$

oder$-C(O)C_6H_5$.

In R' ist $C_3$- bis $C_7$-Cycloalkyl beispielweise Cyclopropyl, Cyclobutyl, Cyclopentyl, Cyclohexyl oder Cycloheptyl.

In R' bedeutet substituiertes Phenyl, durch $C_1$- bis $C_6$-Alkyl, $C_1$- bis $C_6$-Alkenyl, $NO_2$, F, Cl, Br, I, $C_1$-$C_6$-Alkoxy, $SCF_3$, $SO_2CF_3$, COOR'', $SO_2X'$, $SO_2NR''_2$ oder $SO_3R''$ substituiertes Phenyl, wobei X' F, Cl oder Br und R'' ein nicht oder teilweise fluoriertes $C_1$- bis $C_6$-Alkyl oder $C_3$- bis $C_7$-Cycloalkyl wie für R' definiert bedeutet, beispielsweise, o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-Nitrophenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

Die Substituenten $R^1$ bis $R^7$ sind jeweils unabhängig voneinander bevorzugt eine geradkettige oder verzweigte Alkylgruppe mit 1 bis 10 C-Atomen. Die Substituenten $R^1$ und $R^2$, $R^3$ und $R^4$ und $R^5$ und $R^6$ in Verbindungen der Formeln (2) und (3) können dabei gleich oder verschieden sein. Besonders bevorzugt sind $R^1$ bis $R^7$ jeweils unabhängig voneinander Methyl, Ethyl, n-Propyl, Isopropyl, n-Butyl, sek.-Butyl, Phenyl oder Cyclohexyl, ganz besonders bevorzugt Methyl, Ethyl, n-Propyl, Isopropyl oder n-Butyl.

Als Substituenten $R^{1'}$ bis $R^{4'}$ von Verbindungen der Formel (4) kommen erfindungsgemäß dabei neben Wasserstoff bevorzugt in Frage: $C_1$- bis $C_{20}$-, insbesondere $C_1$- bis $C_{12}$-Alkylgruppen, und gesättigte oder ungesättigte; d.h. auch aromatische, $C_3$- bis $C_7$-Cycloalkylgruppen, die mit $C_1$- bis $C_6$-Alkylgruppen substituiert sein können, insbesondere Phenyl oder Aryl-$C_1$-$C_6$-alkyl.

Die Substituenten $R^{1'}$ und $R^{4'}$ sind jeweils unabhängig voneinander insbesondere bevorzugt Methyl, Ethyl, Isopropyl, Propyl, Butyl, sek.-Butyl, Pentyl, Hexyl, Octyl, Decyl, Cyclohexyl, Phenyl, Phenylpropyl oder Benzyl. Sie sind ganz besonders bevorzugt Methyl, Ethyl, n-Butyl oder Hexyl. In Pyrrolidinium-, Piperidinium oder Indolinium-Verbindungen sind die beiden Substituenten $R^{1'}$ und $R^{4'}$ bevorzugt unterschiedlich.

Der Substituent $R^{2'}$ oder $R^{3'}$ ist jeweils unabhängig voneinander insbesondere Wasserstoff, Methyl, Ethyl, Isopropyl, Propyl, Butyl, sek.-Butyl, tert.-Butyl, Cyclohexyl, Phenyl oder Benzyl. Besonders bevorzugt ist $R^{2'}$ Wasserstoff, Methyl, Ethyl, Isopropyl, Propyl, Butyl, sek.-Butyl oder tert.-Butyl. Ganz besonders bevorzugt sind $R^{2'}$ und $R^{3'}$ Wasserstoff oder Methyl.

Bevorzugt sind die Alkylgruppen als Substituenten R und $R^1$ bis $R^6$ sowie $R^{1'}$ und $R^{4'}$ der heterocyclischen Kationen der Formel (4) unterschiedlich von der Alkylgruppe des eingesetzten entsprechenden Esters, Trialkylsilylesters oder des gemischten Alkyl-trialkylsilylesters der Phosphor-, Dialkylphosphin- oder Alkylphosphonsäure.

Das erfindungsgemäß hergestellte Onium-Dialkylphosphat, Onium-Dialkylphosphinat, Onium-(O-alkyl)-alkylphosphonat oder Onium-alkyl-phosphonat kann jedoch auch Alkylgruppen im Kation haben, die zu der Alkylgruppe im

Ester gleich sind, die jedoch erfindungsgemäß nicht durch Alkylierung eingeführt wurden. Der Focus liegt dann auf der einfachen Reaktionsführung und des besonders niedrigen Halogenid-Gehalts im Endprodukt.

HetN$^+$ der Formel (4) ist bevorzugt

Imidazolium

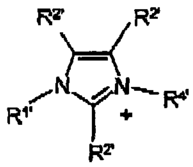

,

Pyrazolium

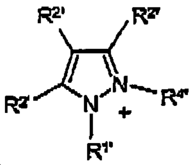

,

Pyrrolidinium

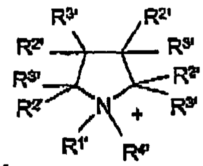

,

Pyridinium

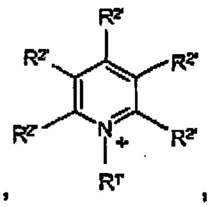

,

Pyrimidinium

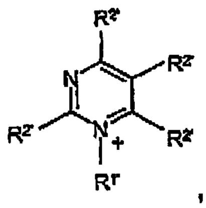

,

Piperidinium

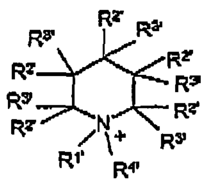

oder

Indolinium

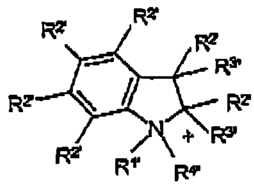

,

wobei die Substituenten $R^{1'}$ bis $R^{4'}$ jeweils unabhängig voneinander eine zuvor beschriebene Bedeutung haben.

HetN* ist besonders bevorzugt Imidazolium. Pyrrolidinium oder Pyridinium, wie zuvor definiert, wobei die Substituenten $R^{1'}$ bis $R^{4'}$ jeweils unabhängig voneinander eine zuvor beschriebene Bedeutung haben.

Als Ester einer Phosphorsäure wird bevorzugt ein entsprechender Ester mit geradkettigen oder verzweigten Alkylgruppen mit 1-8 C-Atomen, bevorzugt mit 1-4 C-Atomen eingesetzt, die jeweils unabhängig voneinander sind. Vorzugsweise sind die Alkylgruppen des Esters gleich.

Die eingesetzten Alkylester einer Phosphorsäure sind in der Regel kommerziell erhältlich oder können nach Syntheseverfahren hergestellt werden, wie sie aus der Literatur, z.B. in den Standardwerken wie Houben-Weyl. Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Beispiele für Trialkylphosphorsäureester sind Trimethylphosphat, Triethylphosphat, Tripropylphosphat, Tributylphosphat, Tripentylphosphat, Trihexylphosphat, Triheptylphosphat oder Trioctylposphat. Besonders bevorzugt wird Trimethylphosphat oder Triethylphosphat eingesetzt.

Als Trialkylsilylester oder gemischte Alkyl-trialkisilylester der Phosphorsäure können Tris(trialkylsilyl)phosphat Bis(trialkylsilyl)alkylphosphat oder Trialkylsilyldialkylphosphat, eingesetzt werden, wobei die Alkylgruppen linear oder verzweigt sein können mit 1 bis 8 C-Atomen, bevorzugt mit 1 bis 4 C-Atomen. Die Alkylgruppen der Trialkylsilylgruppe sind bevorzugt gleich und haben 1 bis 4 C-Atome.

Beispiele der zuvor genannten Ester sind Tris(trimethylsilyl)phosphat, Bis(trimethylsilyl)methylphosphat, Bis (trimethylsilyl)ethylphosphat oder Trimethylsilyldirnethylphosphat.

Die eingesetzten Trialkylsilylester oder gemischten Ester einer Phosphorsäure, wie zuvor beschrieben sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Vertag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Ein allgemeines Schema fasst das erfindungsgemäße Verfahren zusammen:

$[PR_4]^+$ $Hal^-$ (1) oder $[(R^1R^2N)\text{-}C(=SR^7)(NR^3R^4)]^+$ $Hal^-$ (2)

oder $[C(NR^1R^2)(NR^3R^4)(NR^5R^6)]^+$ $Hal^-$ (3)

oder $[HetN]^+$ $Hal^-$ (4)

+ [Trialkylphosphat] oder Trialkylsilylester oder gemischte Ester dieser Säure

$[PR_4]^+$[Säureanion]$^-$ (5) oder

$[(R^1R^2N)\text{-}C(=SR^7)(NR^3R^4)]^+$ [Säureanion]$^-$ (6) oder

$[C(NR^1R^2)(NR^3R^4)(NR^5R^6)]^+$ [Säureanion]$^-$ (7) oder

$[HetN]^+$ [Säureanion]$^-$ (8)

+ Alkyl-Hal oder $(Alkyl)_3Si$-Hal

Die Substituenten R, $R^1$ bis $R^7$ und $MetN^+$ der Verbindungen der Formeln (1) bis (8) entsprechen den Bedeutungen, wie zuvor beschrieben. [Säureanion]- bedeutet das entsprechende Anion aus dem eingesetzten Ester nach Abspaltung einer Alkylgruppe, beispielsweise $[(Alkyl\text{-}O)_2P(O)O]^-$.

Die Reaktion wird erfindungsgemäß bei Temperaturen zwischen 20° und 100°C, bevorzugt bei 80° bis 100°, besonders bevorzugt bei 100°C durchgeführt, wenn Alkylester der entsprechenden Säuren eingesetzt werden. Werden die Trialkylsilylester oder die gemischten Ester der Säuren eingesetzt, findet die Reaktion zwischen 0°C und 30°C, bevorzugt bei Raumtemperatur statt. Es wird kein Lösungsmittel benötigt. Es können jedoch auch Lösungsmittel eingesetzt werden, beispielsweise Dimethoxyethan, Acetonitril, Aceton, Tetrahydrofuran, Dimethylformamid, Dimethylsulfoxid, Dioxan, Propionitril oder Gemische untereinander.

Die Umsetzung wird mit einem maximalen Überschuss bis 20% oder equimolarer Menge an dem entsprechenden Ester der Phosphorsäure durchgeführt.

Die erfindungsgemäße Methode ist auch für die Reinigung von halogenidhaltigen Onium-Salzen mit Dialkylphosphat.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Es versteht sich für den Fachmann von selbst, dass in den vorab und nachfolgend genannten Verbindungen Substituenten wie beispielsweise H, N, O, Cl, F durch die entsprechenden Isotope ersetzt sein können.

Die NMR-Spektren wurden an Lösungen in deuterierten Lösungsmitteln bei 20°C an einem Bruker ARX 400 Spektrometer mit einem 5 mm Breitbandkopf $^1H$/BB mit Deuterium Lock gemessen, falls nicht in den Beispielen angegeben. Die Messfrequenzen der verschiedenen Kerne sind: $^1H$: 400,13 MHz und $^{19}F$: 376,50 MHz. $^{91}P$-Spektren wurden an einem Bruker Avance 250 Spektromether gemessen mit der Meßfrequenz 101,26 MHz. Die Methode der Referenzierung wird bei jedem Spektrum bzw. bei jedem Datensatz separat angegeben.

Beispiel 1:

Synthese von 1-Butyl-3-methylimidazolium Dimethylphosphat

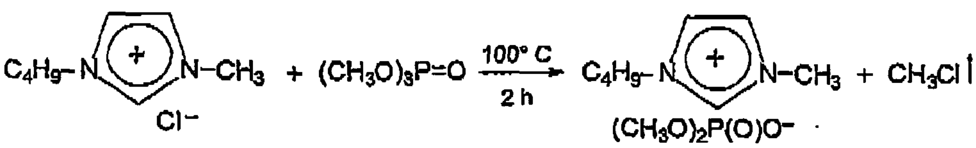


Eine Mischung von 3.25 g (18.6 mmol) 1-Butyl-3-methylimidazolium chlorid und 2.61 g (18.6 mmol) Trimethylphosphat werden bei 100°C Ölbadtemperatur für zwei Stunden erhitzt. Der Rückstnd wird bei 100°C (Ölbadtemperatur) und einem Vakuum von 13.3 Pa für eine Stunde getrocknet. Man erhält 4.91 g 1-Butyl-3-methylimidazolium Dimethylphosphat in annähernd quantitativer Ausbeute.

$^{1}H$ NMR (Referenz: TMS; $CD_3CN$), ppm: 0.88 t ($CH_3$); 1.27 m ($CH_2$); 1.79 m ($CH_2$); 3.37 d ($OCH_3$); 3.87 s ($CH_3$); 4.18 t ($CH_2$); 7.57 m (CH); 7.60 m (CH): 10.03 br. s. (CH); $^{3}J_{H,H}$ = 7.4 Hz; $^{3}J_{H,H}$ = 7.2 Hz; $^{3}J_{P,H}$ = 10.4 Hz.

$^{31}P$ {$^{1}H$} NMR (Referenz: 85% $H_3PO_4$ - extern; $CD_3CN$), ppm: 1.71.

Beispiel 2:

Synthese von Tetraethylphosphonium Dimethylphosphat

$$(C_2H_5)_4P^+\ Cl^- + (CH_3O)_3P{=}O \xrightarrow[3\ h]{100°\ C} [(C_2H_5)_4P]^+ [(CH_3O)_2P(O)O]^- + CH_3Cl\uparrow$$

Eine Mischung von 0.50 g (2.74 mmol) Tetraethylphosphoniumchlorid und 0.46 g (3.28 mmol) Trimethylphosphat werden für 3 Stunden auf 100°C erhitzt. Der Rückstand wird anschließend bei 100°C 30 Minuten lang im Vakuum bei 13.3 Pa behandelt. Man erhält 0.74 g Tetraethylphosphonium Dimethylphosphat. Die Ausbeute ist annähernd quantitativ. Smp. 48-49°C.

$^{1}H$ NMR (Referenz: TMS; $CD_3CN$), ppm: 1.19 d,t ($4CH_3$); 2.26 m ($4CH_2$); 3.37 d ($20CH_3$); $^{3}J_{H,P}$ = 10.3 Hz; $^{3}J_{H,P}$ = 18.0 Hz; 3 $J_{H,H}$ = 7.7 Hz. $^{31}P$ NMR (Referenz: 85% $H_3PO_4$ - extern: $CD_3CN$), ppm: 0.4 hep (1 P); 39.5 m (1 P).

**Patentansprüche**

1. Verfahren zur Herstellung von Onium-Salzen mit Dialkylphosphat-Anionen durch Umsetzung eines Onium-Halogenids mit einem Phosphorsäuretrialkylester oder einem Trialkylsilylester oder einem gemischten Alkyl-trialkylsilylester der Phosphorsäure, wobei der eingesetzte Ester das Anion des eingesetzten Onium-Halogenids alkyliert.

2. Verfahren nach Anspruch 1, **dadurch gekennzeichnet, dass** das Halogenid ein Phosphoniumhalogenid, Thiouroniumhalogenid, Guanidiniumhalogenid oder Halogenid mit heterocyclischem Kation ist.

3. Verfahren nach Anspruch 1 oder 2, **dadurch gekennzeichnet, dass** das Halogenid der Formel (1) entspricht,

$[PR_4]^+ Hal^-$ (1),

wobei

Hal Cl, Br oder I und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise oder vollständig mit -F substituiert sein können, wobei jedoch nicht alle vier oder drei R vollständig mit F substituiert sein dürfen,
und wobei ein oder zwei nicht benachbarte und nicht $\alpha$- oder $\omega$-ständige Kohlenstoffatome des R, durch Atome und/oder Atomgruppierungen ausgewählt aus der Gruppe -O-, -S-, -S(O)- oder $-SO_2-$ ersetzt sein können.

4. Verfahren nach nach Anspruch 1 oder 2, **dadurch gekennzeichnet, dass** das Halogenid der Formel (2) entspricht,

$[(R^1R^2N)-C(=SR^7)(NR^3R^4)]^+ Hal^-$ (2),

wobei

Hal Cl, Br oder I und
$R^1$ bis $R^7$ jeweils unabhängig voneinander

Wasserstoff oder CN, wobei Wasserstoff für $R^7$ ausgeschlossen wird, geradkettiges oder verzweigtes Alkyl mit 1 bis 20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten $R^1$ bis $R^7$ teilweise oder vollständig mit -F substituiert sein können, wobei jedoch nicht alle Substituenten an einem N-Atom vollständig mit F substituiert sein dürfen, wobei die Substituenten $R^1$ bis $R^7$ paarweise durch Einfach- oder Doppelbindung miteinander verbunden sein können und wobei ein oder zwei nicht benachbarte, nicht direkt am Heteroatom gebundene und nicht ω-ständige Kohlenstoffatome der Substituenten $R^1$ bis $R^6$ durch Atome und/oder Atomgruppierungen ausgewählt aus der Gruppe -O-, -S-, -S(O)- oder $-SO_2-$ ersetzt sein können.

5. Verfahren nach nach Anspruch 1 oder 2, **dadurch gekennzeichnet, dass** das Halogenid der Formel (3) entspricht,

$[C(NR^1R^2)(NR^3R^4)(NR^5R^6)]^+ Hal^-$ (3),

wobei,

Hal Cl, Br oder I und
$R^1$ bis $R^6$ jeweils unabhängig voneinander
Wasserstoff oder CN,
geradkettiges oder verzweigtes Alkyl mit 1 bis 20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,

wobei ein oder mehrere der Substituenten $R^1$ bis $R^6$ teilweise oder vollständig mit -F substituiert sein können, wobei jedoch nicht alle Substituenten an einem N-Atom vollständig mit F substituiert sein dürfen, wobei die Substituenten $R^1$ bis $R^6$ paarweise durch Einfach- oder Doppelbindung miteinander verbunden sein können und wobei ein oder zwei nicht benachbarte, nicht direkt am Heteroatom gebundene und nicht ω-ständige Kohlenstoffatome der Substituenten $R^1$ bis $R^6$ durch Atome und/oder Atomgruppierungen ausgewählt aus der Gruppe -O-, -S-, -S(O)- oder $-SO_2-$ ersetzt sein können.

6. Verfahren nach nach Anspruch 1 oder 2, **dadurch gekennzeichnet, dass** das Halogenid der Formel (4) entspricht,

$[HetN]^+ Hal^-$ (4)

wobei

| | |
|---|---|
| Hal | Cl, Br oder I und |
| $HetN^+$ | ein heterocyclisches Kation, ausgewählt aus der Gruppe |

Imidazolium

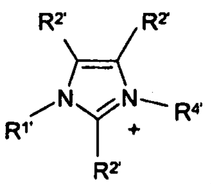

,

1H-Pyrazolium

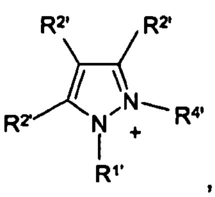

,

3H-Pyrazolium

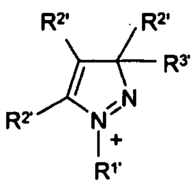

,

4H-Pyrazolium

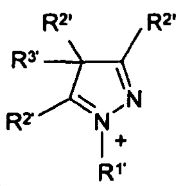

,

1-Pyrazolinium

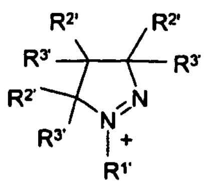

,

2-Pyrazolinium

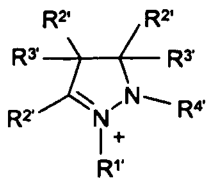

,

3-Pyrazolinium

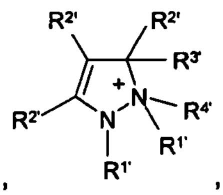

,

2,3-Dihydro-Imidazolinium

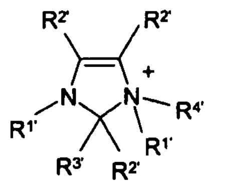

,

4,5-Dihydro-Imidazolinium

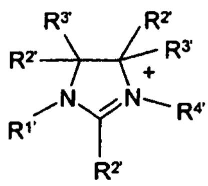

,

2,5-Dihydro-Imidazolinium

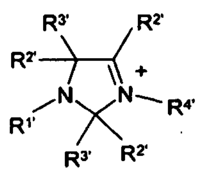

,

Pyrrolidinium

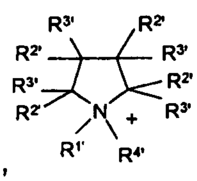

,

1,2,4-Triazolium

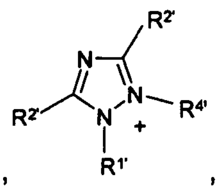

,

1,2,4-Triazolium

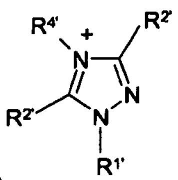

,

1,2,3-Triazolium

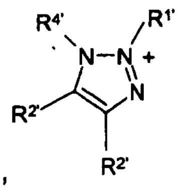

,

1,2,3-Triazolium

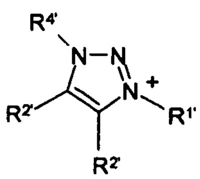

,

Pyridinium

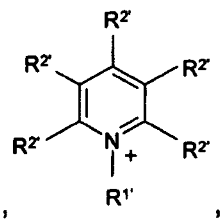

,

Pyridazinium

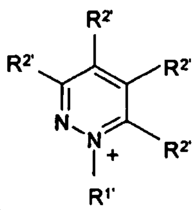

,

Pyrimidinium

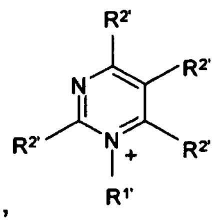

,

Piperidinium

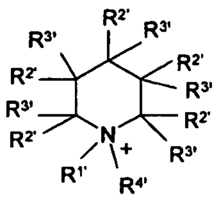

,

Morpholinium

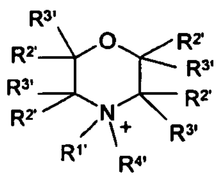

,

Pyrazinium

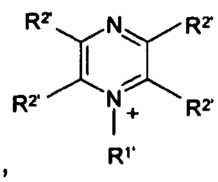

,

Thiazolium

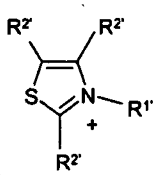

,

Oxazolium

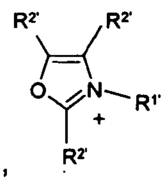

,

Indolium

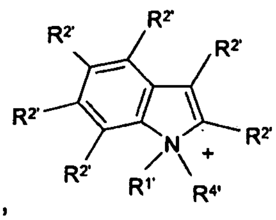

,

Chinolinium

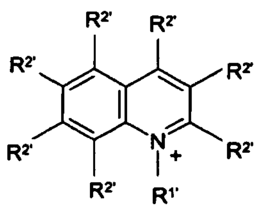

,

Isochinolinium

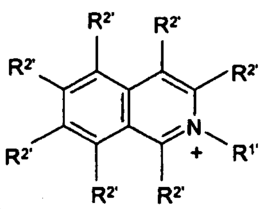

,

Chinoxalinium

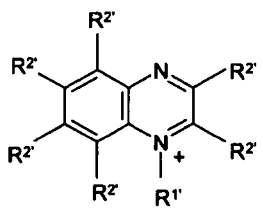

oder

Indolinium

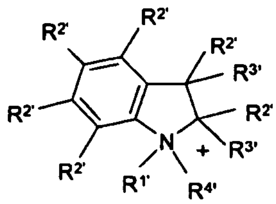

bedeutet, wobei die Substituenten

$R^{1'}$ bis $R^{4'}$ jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7C-Atomen, das mit Alkylgruppen mit 1-6C-Atomen substituiert sein kann oder Aryl-$C_1$-$C_6$-alkyl bedeutet,
wobei die Substituenten $R^1$, und $R^4$, teilweise oder vollständig mit F substituiert sein können, wobei jedoch nicht gleichzeitig $R^{1'}$ und $R^{4'}$ CN sind oder vollständig mit F substituiert sein dürfen,
wobei die Substituenten $R^{2'}$ und $R^{3'*}$ teilweise oder vollständig mit Halogenen oder teilweise mit $NO_2$ oder CN substituiert sein dürfen und wobei ein oder zwei nicht benachbarte, nicht direkt am Heteroatom gebundene und nicht ω-ständige Kohlenstoffatome der Substituenten $R^{1'}$ bis $R^{4'}$, durch Atome und/oder Atomgruppierungen ausgewählt aus der Gruppe -O-, -S-, -S(O)- oder -$SO_2$- ersetzt sein können.

7. Verfahren nach einem oder mehreren der Ansprüche 1 bis 6 **dadurch gekennzeichnet, dass** die Reaktion der Alkylester der Phosphorsäure bei Temperaturen von 20°C bis 100°C durchgeführt wird.

8. Verfahren nach einem oder mehreren der Ansprüche 1 bis 6, **dadurch gekennzeichnet, dass** die Reaktion der Trialkylsilylester der Phosphorsäure bei Temperaturen von 0°C bis 30°C durchgeführt wird.

9. Verfahren nach einem oder mehreren der Ansprüche 1 bis 8, **dadurch gekennzeichnet, dass** die Reaktion ohne Lösungsmittel durchgeführt wird.

10. Verwendung des Verfahrens nach einem oder mehreren der Ansprüche 1 bis 9 zur Aufreinigung von durch Onium-Halogenide verunreinigten ionischen Flüssigkeiten mit Dialkylphosphat-Anionen.

## Claims

1. Process for the preparation of onium salts with dialkylphosphate anions by reaction of an onium halide with a trialkyl phosphate or a trialkylsilyl ester or a mixed alkyl trialkylsilyl ester of phosphoric acid, where the ester employed alkylates the anion of the onium halide employed.

2. Process according to Claim 1, **characterised in that** the halide is a phosphonium halide, thiouronium halide, guanidinium halide or halide with a heterocyclic cation.

3. Process according to Claim 1 or 2, **characterised in that** the halide conforms to the formula (1)

$$[PR_4]^+ Hal^- \qquad (1),$$

where

Hal denotes Cl, Br or I and
R in each case, independently of one another, denotes

H, where all substituents R cannot simultaneously be H,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more R may be partially or fully substituted by -F, but where all four or three R cannot be fully substituted by F,
and where, in the R, one or two non-adjacent carbon atoms which are not in the α- or ω-position may be replaced by atoms and/or atom groups selected from the group -O-, -S-, -S(O)- or $-SO_2-$.

4. Process according to Claim 1 or 2, **characterised in that** the halide conforms to the formula (2)

$$[(R^1R^2N)\text{-}C(=SR^7)(NR^3R^4)]^+ \; Hal^- \qquad (2),$$

where

Hal denotes Cl, Br or I and
$R^1$ to $R^7$ each, independently of one another, denote hydrogen or CN, where hydrogen is excluded for $R^7$,
straight-chain or branched alkyl having 1 to 20C atoms, straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents $R^1$ to $R^7$ may be partially or fully substituted by -F, but where all substituents on an N atom must not be fully substituted by F,
where the substituents $R^1$ to $R^7$ may be bonded to one another in pairs by a single or double bond
and where, in the substituents $R^1$ to $R^7$, one or two non-adjacent carbon atoms which are not bonded directly to the heteroatom and are not in the ω-position may be replaced by atoms and/or atom groups selected from the group -O-, -S-, -S(O)- or $-SO_2-$.

5. Process according to Claim 1 or 2, **characterised in that** the halide conforms to the formula (3)

$$[C(NR^1R^2)(NR^3R^4)(NR^5R^6)]^+ \; Hal^- \qquad (3),$$

where

Hal denotes Cl, Br or I and
$R^1$ to $R^6$ each, independently of one another, denote hydrogen or CN,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents $R^1$ to $R^6$ may be partially or fully substituted by -F, but where all substituents on an N atom must not be fully substituted by F,
where the substituents $R^1$ to $R^6$ may be bonded to one another in pairs by a single or double bond
and where, in the substituents $R^1$ to $R^6$, one or two non-adjacent carbon atoms which are not bonded directly to the heteroatom and are not in the ω-position may be replaced by atoms and/or atom groups selected from the group -O-, -S-, -S(O)- or $-SO_2-$.

6. Process according to Claim 1 or 2, **characterised in that** the halide conforms to the formula (4)

$$[HetN]^+ \; Hal^- \qquad (4),$$

where

Hal denotes Cl, Br or I and

$HetN^+$ denotes a heterocyclic cation selected from the group

imidazolium

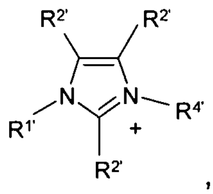,

1H-pyrazolium

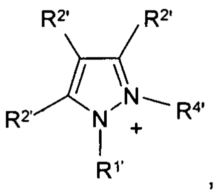,

3H-pyrazolium

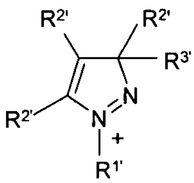,

4H-pyrazolium

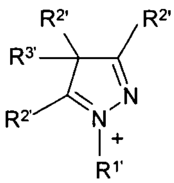,

1-pyrazolinium

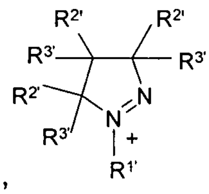,

2-pyrazolinium

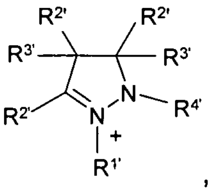,

3-pyrazolinium

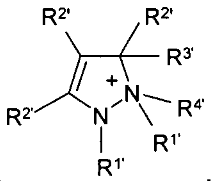,

2,3-dihydroimidazolinium

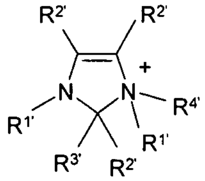,

4,5-dihydroimidazolinium

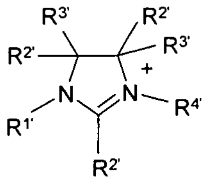,

2,5-dihydroimidazolinium

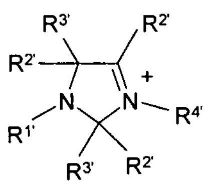,

pyrrolidinium

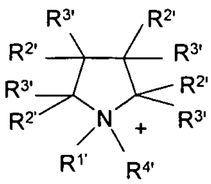,

1,2,4-triazolium

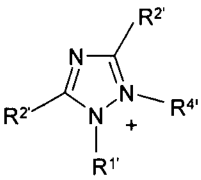,

1,2,4-triazolium

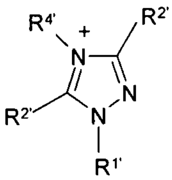,

1,2,3-triazolium

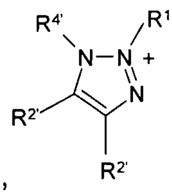,

1,2,3-triazolium

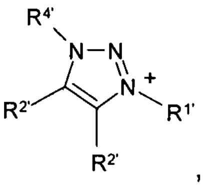,

pyridinium

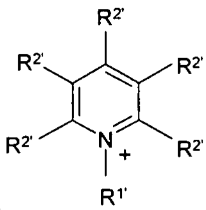,

pyridazinium

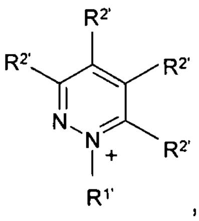,

pyrimidinium

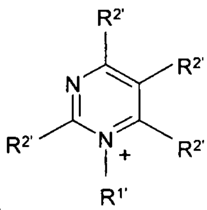,

piperidinium

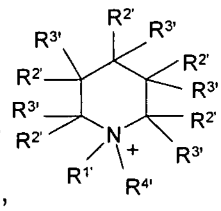,

morpholinium

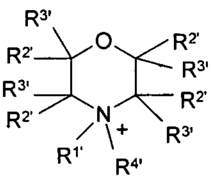,

pyrazinium

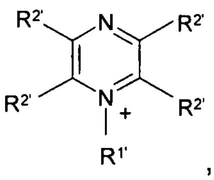,

thiazolium

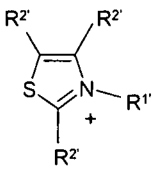,

oxazolium

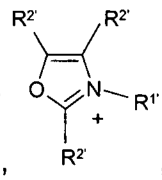,

indolium

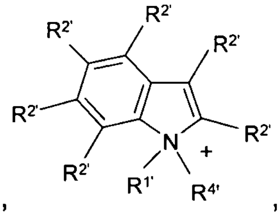,

quinolinium, isoquinolinium, quinoxalinium

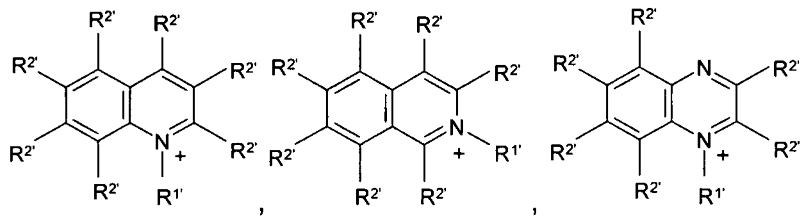

or

indolinium

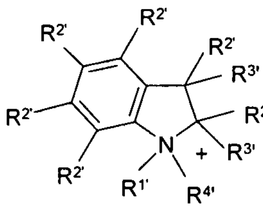

,

where the substituents

$R^1$, to $R^{4'}$ each, independently of one another, denote hydrogen or CN,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
dialkylamino containing alkyl groups having 1-4 C atoms, but which is not bonded to the heteroatom of the heterocycle,
saturated, partially or fully unsaturated cycloalkyl having 3-7 C atoms, which may be substituted by alkyl groups having 1-6 C atoms, or aryl-$C_1$-$C_6$-alkyl,
where the substituents $R^{1'}$ and $R^{4'}$ may be partially or fully substituted by F, but where $R^{1'}$ and $R^{4'}$ cannot simultaneously be CN or fully substituted by F,
where the substituents $R^{2'}$ and $R^{3'}$ may be partially or fully substituted by halogens or partially substituted by $NO_2$ or CN
and where, in the substituents $R^{1'}$ to $R^{4'}$, one or two non-adjacent carbon atoms which are not bonded directly to the heteroatom and are not in the ω-position may be replaced by atoms and/or atom groups selected from the group -O-, -S-, -S(O)- or $-SO_2-$.

**7.** Process according to one or more of Claims 1 to 6, **characterised in that** the reaction of the alkyl esters of phosphoric acid is carried out at temperatures of 20°C to 100°C.

**8.** Process according to one or more of Claims 1 to 6, **characterised in that** the reaction of the trialkylsilyl esters of phosphoric acid is carried out at temperatures of 0°C to 30°C.

**9.** Process according to one or more of Claims 1 to 8, **characterised in that** the reaction is carried out without a solvent.

**10.** Use of the process according to one or more of Claims 1 to 9 for the purification of ionic liquids with dialkylphosphate anions which are contaminated by onium halides.

## Revendications

**1.** Procédé de préparation de sels d'onium avec des anions dialkylphosphate par la réaction d'un halogénure d'onium avec un phosphate trialkylé ou un ester trialkylsilylé ou un ester mixte d'alkyle et de trialkylsilyle de l'acide phosphorique, où l'ester employé alkyle l'anion de l'halogénure d'onium employé.

2. Procédé selon la revendication 1, **caractérisé en ce que** l'halogénure est un halogénure de phosphonium, un halogénure de thiouronium, un halogénure de guanidinium ou un halogénure avec un cation hétérocyclique.

3. Procédé selon la revendication 1 ou 2, **caractérisé en ce que** l'halogénure répond à la formule (1)

$$[PR_4]^+ Hal^- \qquad (1),$$

où

Hal désigne Cl, Br ou I est
R dans chaque cas, indépendamment les uns des autres, désigne
H, où tous les substituants R ne peuvent pas simultanément être H, alkyle à chaîne linéaire ou ramifiée ayant 1-20 atomes de C,
alcényle à chaîne linéaire ou ramifiée ayant 2-20 atomes de C et une ou plusieurs double liaisons,
alcynyle à chaîne linéaire ou ramifiée ayant 2-20 atomes de C et une ou plusieurs triple liaisons,
cycloalkyle saturé ou partiellement ou totalement insaturé ayant 3-7 atomes de C, pouvant être substitué par des groupements alkyle ayant 1-6 atomes de C,
où un ou plusieurs R peuvent être partiellement ou totalement substitués par -F, mais où tous les quatre ou trois R ne peuvent pas être totalement substitués par F,
et où, dans les R, un ou deux atomes de carbone non adjacents qui ne sont pas en position $\alpha$ ou $\omega$ peuvent être remplacés par des atomes et/ou des groupes d'atomes sélectionnés dans le groupe -O-, -S-, -S(O)- ou $-SO_2-$.

4. Procédé selon la revendication 1 ou 2, **caractérisé en ce que** l'halogénure répond à la formule (2)

$$[(R^1R^2N)\text{-}C(=SR^7)(NR^3R^4)]^+ Hal^- \qquad (2),$$

où

Hal désigne Cl, Br ou I est
$R^1$ à $R^7$ indépendamment les uns des autres, désignent chacun hydrogène ou CN, où hydrogène est exclu pour $R^7$,
alkyle à chaîne linéaire ou ramifiée ayant 1 à 20 atomes de C, alcényle à chaîne linéaire ou ramifiée ayant 2-20 atomes de C et une ou plusieurs double liaisons,
alcynyle à chaîne linéaire ou ramifiée ayant 2-20 atomes de C et une ou plusieurs triple liaisons,
cycloalkyle saturé ou partiellement ou totalement insaturé ayant 3-7 atomes de C, pouvant être substitué par des groupements alkyle ayant 1-6 atomes de C,
où un ou plusieurs parmi les substituants $R^1$ à $R^7$ peuvent être partiellement ou totalement substitués par -F, mais où tous les substituants sur un atome de N ne doivent pas être totalement substitués par F,
où les substituants $R^1$ à $R^7$ peuvent être reliés les uns aux autres par paires via une liaison simple ou double,
et où, dans les substituants $R^1$ à $R^7$, un ou deux atomes de carbone non adjacents qui ne sont pas reliés directement à l'hétéroatome et ne sont pas en position $\omega$ peuvent être remplacés par des atomes et/ou des groupes d'atomes sélectionnés dans le groupe -O-, -S-, -S(O)- ou $-SO_2-$.

5. Procédé selon la revendication 1 ou 2, **caractérisé en ce que** l'halogénure répond à la formule (3)

$$[C(NR^1R^2)(NR^3R^4)(NR^5R^6)]^+ Hal^- \qquad (3),$$

où

Hal désigne Cl, Br ou I est
$R^1$ à $R^6$ indépendamment les uns des autres, désignent chacun hydrogène ou CN,
alkyle à chaîne linéaire ou ramifiée ayant 1 à 20 atomes de C, alcényle à chaîne linéaire ou ramifiée ayant 2-20 atomes de C et une ou plusieurs double liaisons,
alcynyle à chaîne linéaire ou ramifiée ayant 2-20 atomes de C et une ou plusieurs triple liaisons,
cycloalkyle saturé ou partiellement ou totalement insaturé ayant 3-7 atomes de C, pouvant être substitué par des groupements alkyle ayant 1-6 atomes de C,

où un ou plusieurs parmi les substituants $R^1$ à $R^6$ peuvent être partiellement ou totalement substitués par -F, mais où tous les substituants sur un atome de N ne doivent pas être totalement substitués par F, où les substituants $R^1$ à $R^6$ peuvent être reliés les uns aux autres par paires via une liaison simple ou double, et où, dans les substituants $R^1$ à $R^6$, un ou deux atomes de carbone non adjacents qui ne sont pas reliés directement à l'hétéroatome et ne sont pas en position w peuvent être remplacés par des atomes et/ou des groupes d'atomes sélectionnés dans le groupe -O-, -S-, -S(O)- ou $-SO_2-$.

**6.** Procédé selon la revendication 1 ou 2, **caractérisé en ce que** l'halogénure répond à la formule (4)

$$[HetN]^+ Hal^- \quad (4),$$

où

Hal désigne Cl, Br ou I est

$HetN^+$ désigne un cation hétérocyclique sélectionné dans le groupe

imidazolium

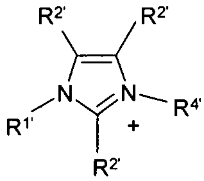

,

1H-pyrazolium

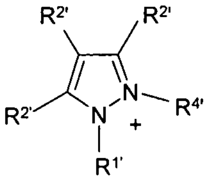

,

3H-pyrazolium

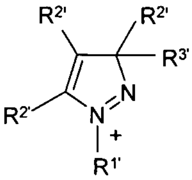

,

4H-pyrazolium

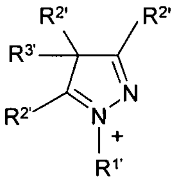

,

1-pyrazolinium

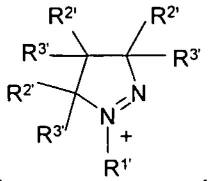

,

2-pyrazolinium

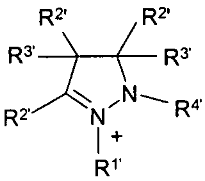

,

3-pyrazolinium

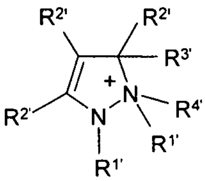

,

2,3-dihydroimidazolinium

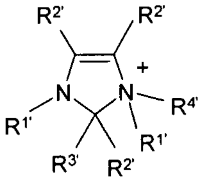

,

4,5-dihydroimidazolinium

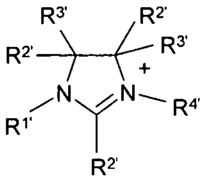

,

2,5-dihydroimidazolinium

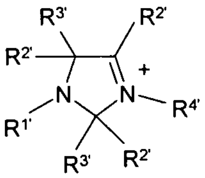

,

pyrrolidinium

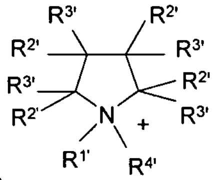

,

1,2,4-triazolium

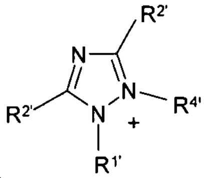

,

1,2,4-triazolium

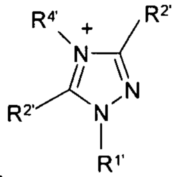

,

1,2,3-triazolium

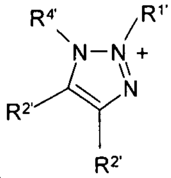

,

1,2,3-triazolium

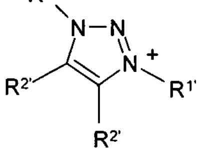

,

pyridinium

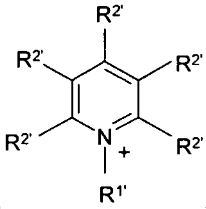

,

pyridazinium

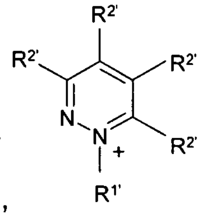

,

pyrimidinium

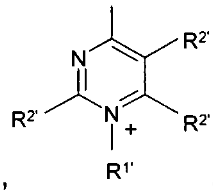

,

pipéridinium

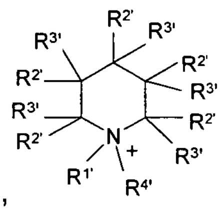

,

morpholinium

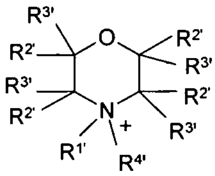

,

pyrazinium

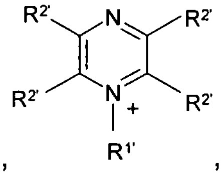

,

thiazolium

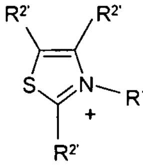

,

oxazolium

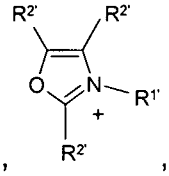

,

indolium

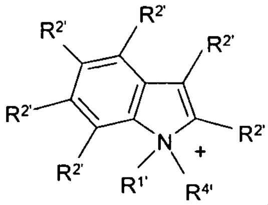

,

quinoléinium

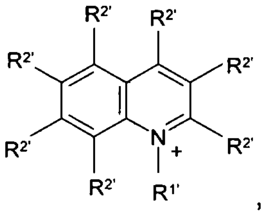

,

isoquinoléinium

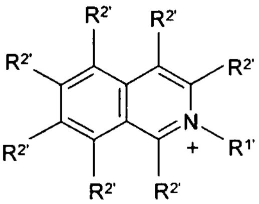

,

quinoxalinium

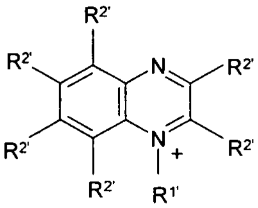

ou

indolinium

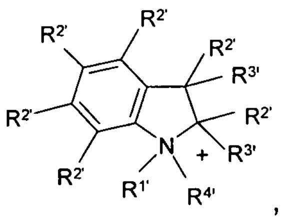

,

où les substituants

$R^1$, à $R^4$, indépendamment les uns des autres, désignent chacun hydrogène ou CN,
alkyle à chaîne linéaire ou ramifiée ayant 1-20 atomes de C,
alcényle à chaîne linéaire ou ramifiée ayant 2-20 atomes de C et une ou plusieurs double liaisons,
alcynyle à chaîne linéaire ou ramifiée ayant 2-20 atomes de C et une ou plusieurs triple liaisons,
dialkylamino contenant des groupements alkyle ayant 1-4 atomes de C, mais qui n'est pas relié à l'hétéroatome de l'hétérocycle,
cycloalkyle saturé ou partiellement ou totalement insaturé ayant 3-7 atomes de C, pouvant être substitué par des groupements alkyle ayant 1-6 atomes de C, ou aryl-$C_1$-$C_6$-alkyle,
où les substituants $R^1$, et $R^4$, peuvent être partiellement ou totalement substitués par F, mais où $R^{1'}$ et $R^{4'}$ ne peuvent pas simultanément être CN ou totalement substitués par F,
où les substituants $R^{2'}$ et $R^{3'}$ peuvent être partiellement ou totalement substitués par des halogènes ou partiellement substitués par $NO_2$ ou CN et où, dans les substituants $R^{1'}$ à $R^{4'}$, un ou deux atomes de carbone non adjacents qui ne sont pas reliés directement à l'hétéroatome et ne sont pas en position w peuvent être remplacés par des atomes et/ou des groupes d'atomes sélectionnés dans le groupe -O-, -S-, -S(O)- ou -$SO_2$-.

**7.** Procédé selon l'une quelconque des revendications 1 à 6, **caractérisé en ce que** la réaction des alkylesters de l'acide phosphorique est réalisée à des températures allant de 20°C à 100°C.

**8.** Procédé selon l'une quelconque des revendications 1 à 6, **caractérisé en ce que** la réaction des esters trialkylsilylés de l'acide phosphorique est réalisée à des températures allant de 0°C à 30°C.

**9.** Procédé selon l'une quelconque des revendications 1 à 8, **caractérisé en ce que** la réaction est réalisée sans solvant.

**10.** Utilisation du procédé selon l'une quelconque des revendications 1 à 9 pour la purification de liquides ioniques ayant des anions dialkylphosphate contaminés par des halogénures d'onium.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 04094438 A
- WO 2004106288 A


### In der Beschreibung aufgeführte Nicht-Patentliteratur


- Chemistry, Biochemistry and Technology. Elsevier, 1980
- **Jean.** *Bull. Soc. Chim. Fr.,* 1957, 783-785
- **R. Jentzsch et al.** *J. Prakt. Chem.,* 1977, vol. 319, 871-874
- **Sutter et al.** *J. Heterocyclic Chem.,* 1980, vol. 17, 43
- **Zakharov N.A. et al.** *Russ. J. Phys. Chem. (engl trans),* 2001, vol. 75 (11), 1924-1927
- **Camerel, Franck et al.** *Chem. Commun.,* 2003, vol. 15, 1958-1959
- **Houben-Weyl.** Methoden der organischen Chemie. Georg-Thieme-Verlag
- **Richard C. Larock.** Comprehensive Organic Transformations. Wiley-VCH, 1999
- **Houben-Weyl.** Methoden der organischen Chemie. Georg-Thieme-Verlag